Europäisches Patentamt
European Patent Office
Office européen des brevets

(11) Veröffentlichungsnummer: **0 338 987 A1**

(12) # EUROPÄISCHE PATENTANMELDUNG

(21) Anmeldenummer: **89810280.1**

(22) Anmeldetag: **11.04.89**

(51) Int. Cl.[4]: **C 07 D 209/48**
C 07 D 405/12, C 07 F 7/10,
C 07 F 7/18, A 01 N 43/00

(30) Priorität: **20.04.88 CH 1449/88**

(43) Veröffentlichungstag der Anmeldung:
**25.10.89 Patentblatt 89/43**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Pissiotas, Georg, Dr.**
**Am Sonnenrain 71**
**D-7850 Lörrach (DE)**

**Moser, Hans, Dr.**
**Hauptstrasse 67B**
**CH-4312 Magden (CH)**

**Brunner, Hans-Georg, Dr.**
**Wannenstrasse 14**
**CH-4415 Lausen (CH)**

(54) **5-(N-3,4,5,6-Tetrahydrophthalsäureimido)benzoesäurethiolester als selektive Herbizide.**

(57) Neue 5-(N-3,4,5,6-Tetrahydrophthalsäureimido)-benzoesäurethiolester der untenstehenden Formel I haben gute pre- und postemergente selektiv-herbizide Eigenschaften ferner beeinflussen respektive hemmen sie das Pflanzenwachstum. Die Ester entsprechen der Formel I

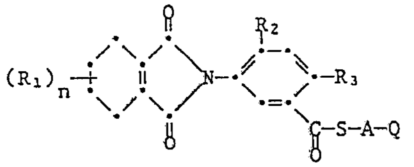

(I),

worin
n Null, eins oder zwei,
$R_1$ $C_1$-$C_3$-Alkyl,
$R_2$ Wasserstoff oder Halogen,
$R_3$ Halogen,
A-Q gemeinsam Wasserstoff oder ein Rest -$CH(COCH_3)CO$-$OR_4$,
A eine geradkettige oder verzweigte $C_1$-$C_4$-Alkylenbrücke, die unsubstituiert oder ein- oder mehrfach durch $C_1$-$C_4$-Alkoxy, $C_1$-$C_4$-Alkylthio oder -CN substituiert ist,
Q Hydroxyl oder einen in der Beschreibung definierten Rest bedeuten.

EP 0 338 987 A1

**Beschreibung**

**5-(N-3,4,5,6-Tetrahydrophthalsäureimido)benzoesäurethiolester als selektive Herbizide**

Die vorliegende Erfindung betrifft neue 5-(N-3,4,5,6-Tetrahydrophthalsäureimido)-benzoesäurethiolester der Formel I mit herbizider und den Pflanzenwuchs regulierender Wirkung, sowie die Herstellung dieser neuen Ester. Ferner betrifft sie Mittel, welche die neuen Verbindungen enthalten sowie deren Verwendung zur selektiven Bekämpfung von Unkräutern oder zur Regulierung des Pflanzenwuchses.

Die neuen 5-(N-3,4,5,6-Tetrahydrophthalsäureimido)-benzoesäurethiolester entsprechen der Formel I

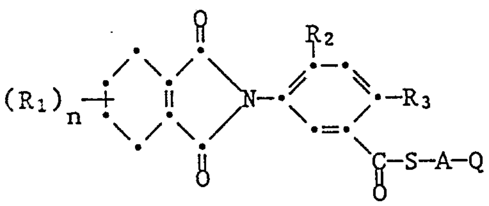

(I),

worin

n Null, eins oder zwei,

$R_1$ $C_1$-$C_3$-Alkyl,

$R_2$ Wasserstoff oder Halogen,

$R_3$ Halogen,

A-Q gemeinsam Wasserstoff oder ein Rest $-CH(COCH_3)COOR_4$,

A eine geradkettige oder verzweigte $C_1$-$C_4$-Alkylenbrücke, die unsubstituiert oder ein- oder mehrfach durch $C_1$-$C_4$-Alkoxy, $C_1$-$C_4$-Alkylthio oder -CN substituiert ist,

Q Hydroxyl, Halogen, -CN, -SCN, $C_2$-$C_6$-Alkenyl, $C_2$-$C_6$-Haloalkenyl, $C_2$-$C_6$-Cyanoalkenyl, $C_2$-$C_4$-Alkinyl oder einen Rest $-C(R_4)=CH-COOR_5$, $-CH[N(R_4)_2]COOR_4$, $-NR_6R_7$, $-COR_8$, $-CON(R_6)SO_2CH_3$, $-N(R_4)CH_2CN$, Diallylcarbamoyl, $-Si(R_{11})_3$, $-COOCH_2Si(CH_3)_2C_1$-$C_6$-alkyl, $-COON=C(R_9)_2$, $-C(R_4)=(OR_{10})_2$, $-PO(OR_{12})-(O)_mR_{12}$, $-CON(R_4)SO_2C_1$-$C_6$- alkyl, $-CON(R_4)-SO_2C_1$-$C_4$-Haloalkyl, $C_1$-$C_6$-Alkylcarbonyl, $C_2$-$C_6$-Alkoxyalkylcarbonyl, einen Benzoyl- oder Benzylcarbonylrest, der unsubstituiert oder durch Halogen, $C_1$-$C_4$-Alkyl, $C_1$-$C_4$-Haloalkyl, $C_1$-$C_4$-Alkoxy, $C_1$-$C_4$-Haloalkoxy, Cyan oder Nitro ein- oder mehrfach substituiert ist, ferner einen Rest $-COOCH(CH_3)CH_2SR_4$, $-CON(R_4)CH_2C(OC_1$-$C_6$-$Alkyl)_2$, $-COO(CH_2)_{n+1}OC_3$-$C_7$-Alkenyl, $-COOC_1$-$C_4$-Cyanoalkyl, $-COO$-$C_1$-$C_4$-$AlkylenN(R_4)_2$ oder $C_1$-$C_8$-Alkanoyloxyrest,

Q bedeutet auch einen Rest $-COOR_{13}$, wenn A durch $C_1$-$C_4$-Alkoxy, $C_1$-$C_4$-Alkylthio oder Cyano substituiert ist,

$R_4$ Wasserstoff, $C_1$-$C_6$-Alkyl oder $C_2$-$C_6$-Alkoxyalkyl,

$R_5$ $C_1$-$C_6$-Alkyl oder $C_1$-$C_6$-Hydroxyalkyl,

$R_6$ und $R_7$ unabhängig voneinander je Wasserstoff, $C_1$-$C_6$-Alkyl, $C_2$-$C_6$-Alkoxyalkyl, $C_3$-$C_6$-Alkenyl, $C_3$-$C_6$-Alkinyl oder $C_3$-$C_6$-Cycloalkyl, 2-Furanylmethyl, 2-Tetrahydrofuranylmethyl, 2-(5-Methyl)-tetrahydrofuranylmethyl, 2-Thienylmethyl,

$R_8$ einen Pyrrolidino-, Piperidino-, Morpholino-, Thiomorpholino-, 4-Methylpiperazino-, Pyrazolino-, Imidazolino- oder 1,2,4-Triazolinrest der unsubstituiert oder ein- oder zweifach durch $C_1$-$C_4$-Alkyl substituiert ist,

$R_9$ $C_1$-$C_6$-Alkyl oder zwei benachbarte $R_9$ bilden zusammen eine $C_2$-$C_6$-Alkylenbrücke

$R_{10}$ $C_1$-$C_4$-Alkyl oder $C_1$-$C_4$-Haloalkyl oder zwei benachbarte $R_{10}$ bilden zusammen eine 1,2-Aethylen-, 1,3-Propylen- oder 1,2-Cyclohexylenbrücke,

$R_{11}$ $C_1$-$C_6$-Alkyl oder $C_1$-$C_6$-Alkoxy,

$R_{12}$ Wasserstoff, $C_1$-$C_6$-Alkyl, $C_1$-$C_6$-Haloalkyl, $C_1$-$C_6$-Cyanoalkyl, $C_3$-$C_4$-Alkenyl oder $C_3$-$C_4$-Alkinyl,

$R_{13}$ $C_1$-$C_{12}$-Alkyl, $C_2$-$C_6$-Alkoxyalkyl, $C_3$-$C_7$-Alkenyl, $C_3$-$C_6$-Alkinyl oder $C_3$-$C_7$-Cycloalkyl und

m 0 oder 1

bedeuten.

In den EP-A 207,894 und 233,151 sind ähnliche Tetrahydrophthalsäureimido-N-benzoesäureester beschrieben. Die vorliegenden Thiolester sind chemisch davon verschieden und zeichnen sich denen gegenüber durch eine spezifischere selektiv-herbizide Wirkung gegen Gräser und Unkräuter in Kulturpflanzen aus.

Speziell gute Wirkung zeigten die Verbindungen der Formel Ia

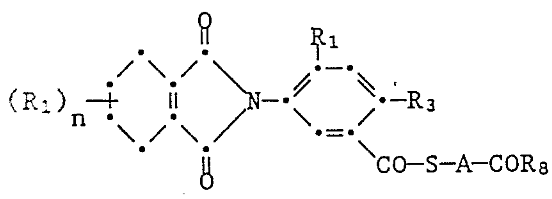

(Ia)

worin n Null, 1 oder 2, $R_1$ $C_3$-$C_6$-Alkyl, $R_2$ Fluor, $R_3$ Chlor oder Brom, A eine $C_1$-$C_4$-Alkylenkette und $R_8$ einen Pyrrolidino-, Piperidino-, Morpholino-, Thiomorpholino-, 4-Methylpiperazino-, Pyrazolino-, Imidazolidino- oder 1,2,4-Triazolinorest bedeutet, der unsubstituiert oder ein- oder zweifach durch $C_1$-$C_4$-Alkyl substituiert ist bedeuten, speziell N-(4-Chlor-2-fluor-5-(2-methylmorpholinocarbonyl)-1-eth-1-yl-thiocarbonylphenyl)-3,4,5,6-tetrahydrophthalimid, N-(4-Chlor-2-fluor-5-pyrrolidinocarbonyl-eth-1-ylthiocarbonylphenyl)-3,4,5,6-tetrahydrophthalimid und N-4-Chlor-2-fluor-5-piperidinocarbonyl-eth-1-ylthiocarbonylphenyl)-3,4,5,6-tetrahydrophthalimid.

Ebenfalls gute Wirkung zeigten die Verbindungen der Formel Ib

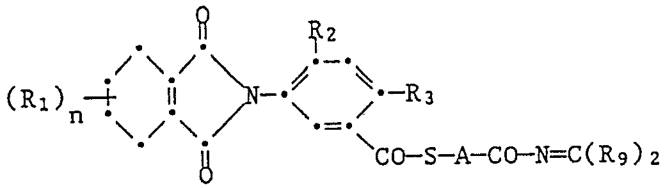

(Ib)

worin n Null, 1 oder 2, $R_1$ $C_3$-$C_6$-Alkyl, $R_2$ Fluor, $R_3$ Chlor oder Brom, A eine $C_1$-$C_4$-Alkylenbrücke und die $R_9$ unabhängig voneinander je $C_1$-$C_6$-Alkyl oder zusammen eine $C_2$-$C_6$-Alkylenbrücke bedeuten, besonders N-(4-Chlor-2-fluor-5-(acetoximcarbonyleth-1-ylthiolcarbonylphenyl)-3,4,5,6-tetrahydrophthalimid.

Weiterhin gute Wirkung zeigten die Verbindungen der Formel Ic

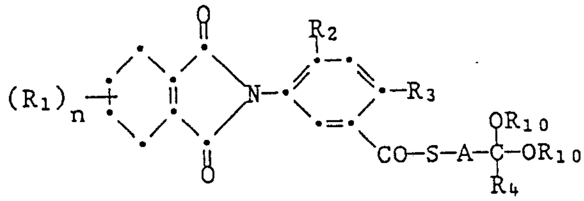

(Ic)

worin n Null, 1 oder 2, $R_1$ $C_3$-$C_6$-Alkyl, $R_2$ Fluor, $R_3$ Chlor oder Brom, A eine $C_1$-$C_4$-Alkylenbrücke, $R_4$ Wasserstoff, $C_1$-$C_6$-Alkyl oder $C_2$-$C_6$-Alkoxyalkyl und die $R_{10}$ unabhängig voneinander je $C_1$-$C_4$-Alkyl, $C_1$-$C_4$-Haloalkyl oder zusammen eine 1,2-Aethylen, 1,3-Propylen- oder 1,2-Cyclohexylenbrücke bedeuten, speziell N-[4-Chlor-5-(1,3-dioxolan-2-ylmethylthiocarbonyl)-2-fluorphenyl]-3,4,5,6-tetrahydrophthalimid.

Ebenso gute Wirkung zeigten die Verbindungen der Formel Id

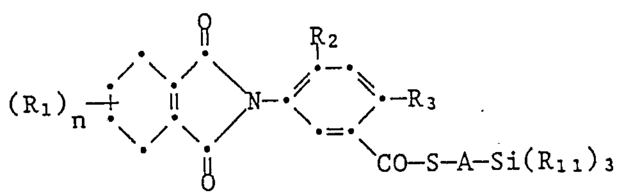

(Id)

worin n Null, 1 oder 2, $R_1$ $C_3$-$C_6$-Alkyl, $R_2$ Fluor, $R_3$ Chlor oder Brom, A eine $C_1$-$C_4$-Alkylenbrücke und die $R_{11}$ unabhängig voneinander je $C_1$-$C_6$-Alkyl oder $C_1$-$C_6$-Alkoxy bedeuten, speziell N-[4-Chlor-2-fluor-5-(triethoxysilyleth-2-ylthiocarbonyl)phenyl]-3,4,5,6-tetrahydrophthalimid und N-[4-Chlor-2-fluor-5-(trimethylsilicylmethylthiocarbonyl)phenyl]-3,4,5,6-tetrahydro-phthalimid.

Gute Wirkung zeigten Verbindungen der Formel Ie ferner die Verbindungen der Formel Ie-Ir

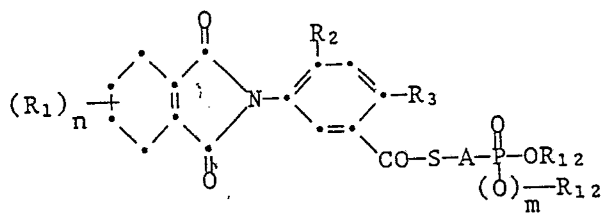

(Ie)

worin n Null, 1 oder 2, $R_1$ $C_3$-$C_6$-Alkyl, $R_2$ Fluor, $R_3$ Chlor oder Brom, A eine $C_1$-$C_4$-Alkylenbrücke, m Null oder 1 und $R_{12}$ unabhängig voneinander je Wasserstoff, $C_1$-$C_6$-Alkyl, $C_1$-$C_6$-Haloalkyl, $C_1$-$C_6$-Cyanoalkyl, $C_3$-$C_4$-Alkenyl oder $C_3$-$C_4$-Alkinyl bedeuten, besonders N-[4-Chlor-5-(diethoxyphosphonsäure-eth-1-ylthiocarbonyl)-2-fluorphenyl]-3,4,5,6-tetrahydrophthalimid.

Verbindungen der Formel If

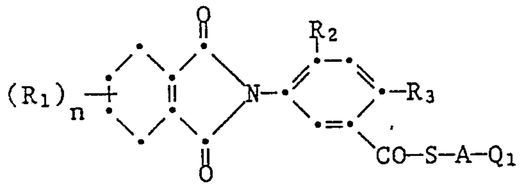

(If)

worin n Null, 1 oder 2, $R_1$ $C_3$-$C_6$-Alkyl, $R_2$ Fluor, $R_3$ Chlor oder Brom, A eine $C_1$-$C_4$-Alkylenbrücke und $Q_1$ Hydroxyl oder Cyan bedeuten, speziell n-[4-Chlor-2-fluor-5-(1-cyano-eth-1-ylthiocarbonyl)phenyl]-3,4,5,6-tetrahydrophthalimid und N-(4-Chlor-2-fluor-5-hydroxyethylthiocarbonylphenyl)-3,4,5,6-tetrahydrophthalimid.

Verbindungen der Formel Ig

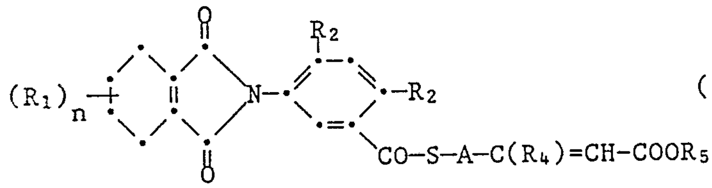

(Ig)

worin A, n, $R_1$, $R_2$, $R_3$, $R_4$ und $R_5$ die oben gegebene Bedeutung haben.

Verbindungen der Formel Ih

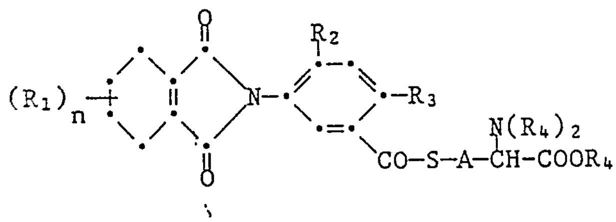

(Ih)

worin A, n, $R_1$, $R_2$, $R_3$ und $R_4$ die unabhängig voneinander, die oben gegebene Bedeutung haben.

Verbindungen der Formel Ii

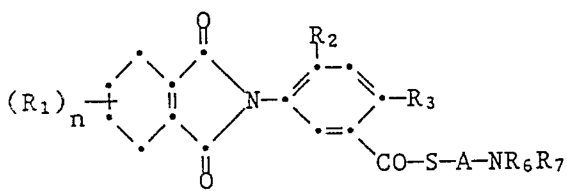

(Ii)

worin A, n, $R_1$, $R_2$, $R_3$, $R_6$ und $R_7$ die oben gegebene Bedeutung haben; speziell N-(4-Chlor-5-dimethylaminoethylthiocarbonyl-2-fluorophenyl)-3,4,5,6-tetrahydrophthalimid.

Verbindungen der Formel Ij

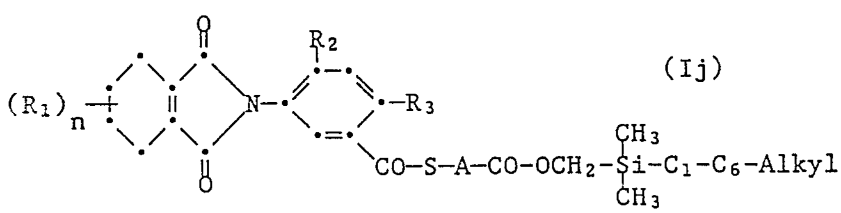

(Ij)

worin A, n, $R_1$, $R_2$ und $R_3$ die oben gegebene Bedeutung haben.

Verbindungen der Formel Ik

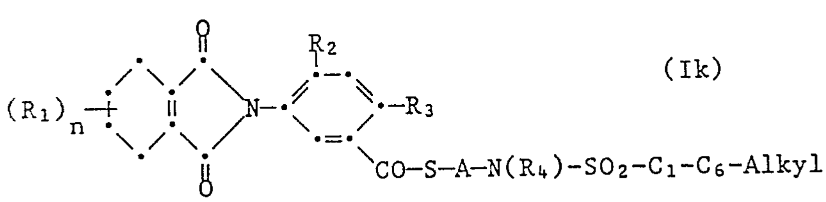

(Ik)

worin A, n, $R_1$, $R_2$ und $R_3$ die oben gegebene Bedeutung haben.

Verbindungen der Formel Il

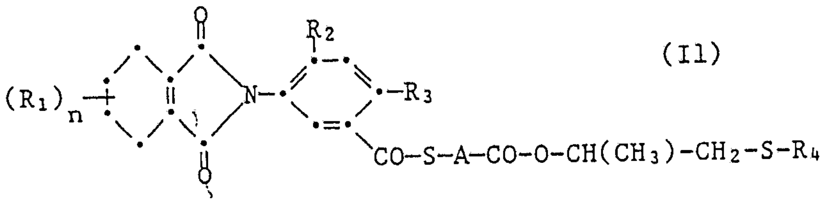

(Il)

worin A, n, $R_1$, $R_2$ und $R_3$ die oben gegebene Bedeutung haben und $R_4$ $C_1$-$C_6$-Alkyl oder $C_2$-$C_6$-Alkoxyalkyl bedeutet, speziell N-[5-Aethylthioprop-2-yloxycarbonyleth-1-ylthiocarbonyl)-4-chlor-2-fluorphenyl]-3,4,5,6-tetrahydrophthalimid.

Verbindungen der Formel Im

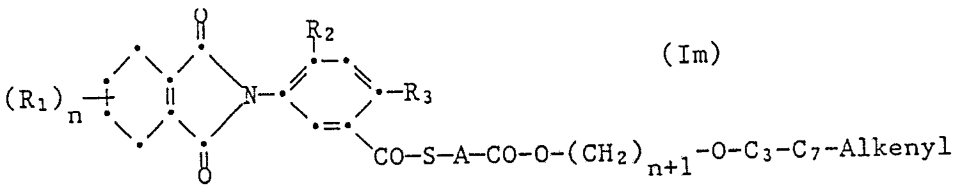

(Im)

worin A, m, $R_1$, $R_2$ und $R_3$ die oben gegebene Bedeutung haben.

Verbindungen der Formel In

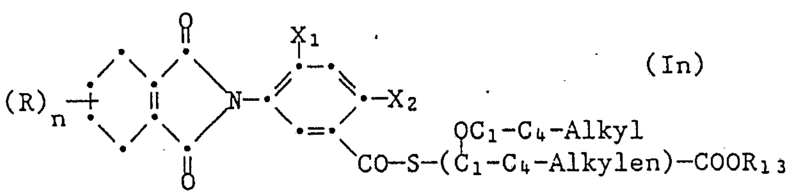

(In)

worin n, $R_1$, $R_2$, $R_3$ und $R_{13}$ die oben gegebene Bedeutung haben, speziell N-[4-Chlor-2-fluor-5-(1′-methoxycarbonyl-1′-methoxy-methylthiocarbonyl)phenyl]-3,4,5,6-tetrahydrophthalimid und N-[4-chlor-2-fluor-5(methoxycarbonyl-2′-methoxymethyl-ethylthiocarbonyl)-phenyl]-3,4,5,6-tetrahydrophthalimid.

Verbindungen der Formel Io

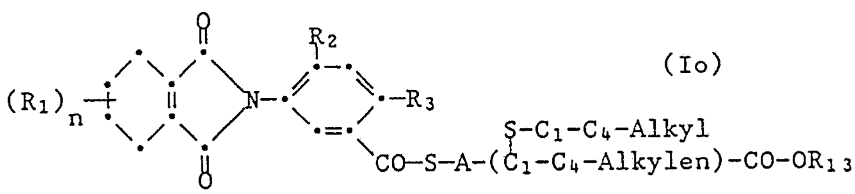

(Io)

worin n, $R_1$, $R_2$, $R_3$ und $R_{13}$ die oben gegebene Bedeutung haben.

Verbindungen der Formel Ip

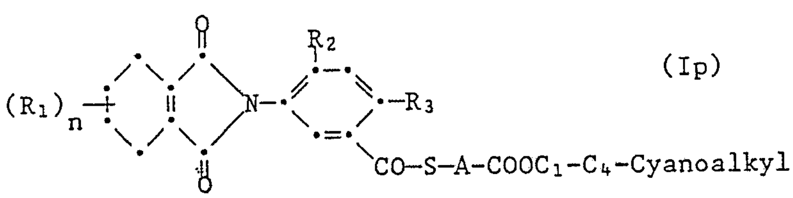

(Ip)

worin A, n, $R_1$, $R_2$ und $R_3$ die oben gegebene Bedeutung haben.

Verbindungen der Formel Iq

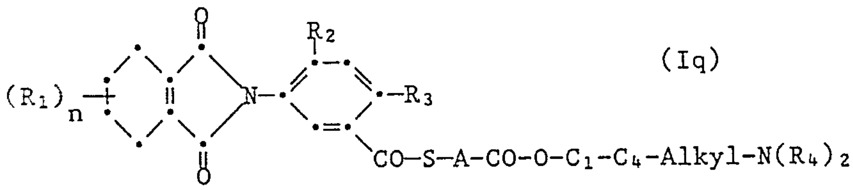

(Iq)

worin A, n, $R_1$, $R_2$, $R_3$ und $R_4$, unabhängig voneinander, die oben gegebene Bedeutung haben.

Verbindungen der Formel Ir

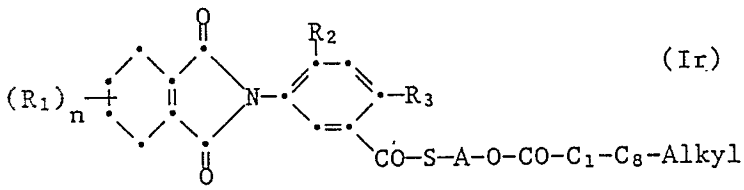

(Ir)

worin A, n, $R_1$, $R_2$ und $R_3$ die oben gegebene Bedeutung haben.

Die Erfindung betrifft auch alle möglichen Stereoisomeren, die in Form von Enantiomeren, Diastereomeren oder deren Gemische vorliegen.

In den obigen Definitionen ist unter Halogen, Fluor, Chlor, Brom oder Iod zu verstehen, vor allem Fluor, Chlor oder Brom.

Der Ausdruck Alkyl allein oder als Teil eines Substituenten umfasst verzweigte und geradkettige Reste. Beispiele sind Methyl, Ethyl, n-Propyl, Isopropyl, n-Butyl, sec. Butyl, tert. Butyl, Isobutyl sowie die höheren Homologen davon, Pentyl, Hexyl, Heptyl, Octyl etc. samt ihren Isomeren. Sinngemäss enthalten Alkanoyle und Cyanalkyle eine zusätzliches Kohlenstoffatom.

Bei den in der obigen Definition erwähnten $C_2$-$C_6$-Alkenyl- oder $C_2$-$C_6$-Alkinylresten handelt es sich ebenfalls um geradkettige oder verzweigte Reste, wobei als Alkenylgruppen insbesondere 2-Propenyl (Allyl), 2-Methyl-2-propenyl (Methallyl), 1-Methyl-2-propenyl, 2-Butenyl und 3-Butenyl und als Alkinylreste 2-Propinyl (Propargyl), 2-Butinyl und 3-Butinyl zu nennen sind. Insbesondere bedeuten in Q und $R_6$ und $R_7$ die Alkenylreste Allyl, Methallyl oder 2-Butenyl und als Alkinylreste insbesondere Propargyl und 2-Butinyl. Sofern die hier genannten ungesättigten Reste über ein Sauerstoffatom an das Molekül gebunden sind, ist der betreffende Rest vorzugsweise über ein gesättigtes Kohlenstoffatom verknüpft.

Die obige Definition von A umfasst Methylen, 1,2-Ethylen und 1,3-Propylen und die durch Substitution von 1 bis 2 Wasserstoffatomen davon abgeleiteten Gruppen, wie z.B. Ethyliden, Isopropyliden (2,2-Propyliden), Benzyliden, 1-Phenyläthyliden, Diphenylmethylen, 1,2-Propylen, 2,3-Butylen, 1,1-Dimethyl-1,2-ethylen, 1,1-Diphenyl-1,2-ethylen, 1,2-Diphenyl-1,2-ethylen, 1-Methyl-1-phenyl-1,2-ethylen, 1,2-Butylen, 1,3-Butylen, 2,2-Dimethyl-1,3-propylen, 1-Methyl-1-phenyl-1,3-propylen, 1,2-Diphenyl-1,3-propylen und 1,3-Diphenyl-1,3-propylen. Von den vorgenannten Bedeutungen von A sind Methylen, 1,2-Ethylen, 1,2-Propylen, 2,3-Butylen und 2,2-Dimethyl-1,3-propylen bevorzugt.

Die in den übrigen Definitionen der Verbindungen der Formel I angegebenen generischen Begriffe umfassen beispielsweise die folgenden spezifischen Einzelsubstituenten, wobei diese Aufzählung keine Einschränkung der Erfindung darstellt:

Mit Halogenalkyl sind die ganz oder teilweise gleich oder unterschiedlich halogensubstituierten Alkyle gemäss der jeweiligen Definitionsbreite gemeint, wie etwa Trifluormethyl, Trichlormethyl, Difluormethyl, Dichlormethyl, 2,2,2-Trifluorethyl und 1,1,1,3,3,3-Hexafluorprop-2-yl.

Mit Alkoxy sind im Rahmen der jeweiligen Definitionsbreite die entsprechenden Alkoxygruppen gemeint; in erster Linie Methoxy und Ethoxy. $C_1$-$C_4$-Alkoxy-$C_2$-$C_4$-alkyloxy, sowie $C_1$-$C_4$-Halogenalkoxy betreffen ebenfalls im Rahmen der jeweiligen Definitionsbreite die entsprechenden Alkylradikale. Hervorzuheben sind Methoxymethyl, Methoxyethyl, Ethoxyethyl, Difluormethoxy, Dichlorfluormethoxy, Trifluormethoxy, Chlordifluormethoxy und Trichlormethoxy.

In den Substituenten, welche aus mehreren Grundelementen zusammengesetzt sind, können die Teilelemente innerhalb der Definitionsbreite frei gewählt werden.

Die Herstellung der erfindungsgemässen N-Phenyl-3,4,5,6-tetrahydrophthalsäureimide der Formel I erfolgt nach an sich bekannten Verfahren.

Die neuen 5-(N-3,4,5,6-Tetrahydrophthalsäureimido)-benzoesäurethiolester der Formel I werden erfindungsgemäss hergestellt, indem man das Anhydrid einer 3,4,5,6-Tetrahydrophthalsäure der Formel II

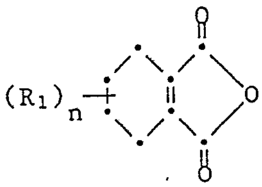

(II)

worin $R_1$ und n die unter der Formel I gegebene Bedeutung haben, in einem inerten organischen Lösungs- oder Verdünnungsmittel, gegebenenfalls in Gegenwart einer geringen Menge eines wasserentziehenden Mittels, mit der äquimolaren Menge eines Derivates einer 3-Aminobenzoesäureester der Formel III umsetzt

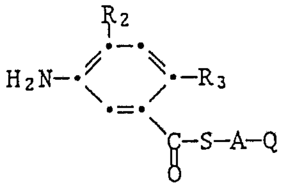

(III),

worin A, $R_2$ und $R_3$ die unter der Formel I gegebene Bedeutung haben.

Als Lösungs- oder Verdünnungsmittel eignen sich für diese Umsetzung höhersiedende Kohlenwasserstoffe, niedere Alkansäuren sowie deren Ester, höhersiedende Ketone und Aether. Als Beispiele seien Toluol, Xylol, Essigsäure, Essigsäureäthylester, Isopropyläther, Tetrahydrofuran, Methyläthylketon, sowie Dimethylformamid erwähnt.

Die Umsetzung findet bei einer Temperatur, die zwischen 0° und 200° liegt, statt.

Zur Beschleunigung der Reaktion kann eine geringe Menge eines wasserentziehenden resp. wasserabsorbierenden Mittels zugegeben werden, wie beispielsweise Schwefelsäure oder einer organischen Sulfonsäure, oder eines Anhydrides, wie Phosphorpentoxid.

Das 3-Aminothiolbenzoesäurederivat der Formel III kann z.B. entsprechend der folgenden Synthese hergestellt werden:

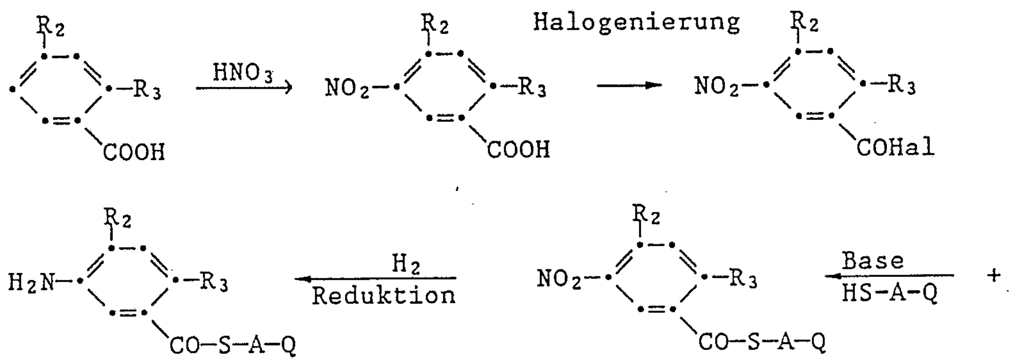


III

In diesen Formeln haben $R_2$, $R_3$, A und Q die unter der Formel I gegebene Bedeutung.

Eine im Phenylkern entsprechend substituierte Benzoesäure wird mit einem Salpetersäuregemisch nitriert und die entstandene 3-Nitrobenzoesäure anschliessend zum Säurehalogenid umgewandelt, z.B. mittels Phosphoroxychlorid oder -bromid, Thionylchlorid oder -bromid oder Sulfurylchlorid oder -bromid.

Das entstandene Säurehalogenid wird dann in Gegenwart der molaren Menge einer Base mit einem Merkaptan der Formel HS-A-Q umgesetzt, wobei A und Q die unter Formel I gegebene Bedeutung haben.

Die 3-Nitrothiolbenzoesäure kann aber auch direkt in Gegenwart einer Base mit einem Alkylhalogenid Hal-A-Q, worin A und Q die unter Formel I gegebene Bedeutung haben und Hal ein Halogenatom, vorzugsweise Chlor oder Brom bedeuten, kondensiert werden.

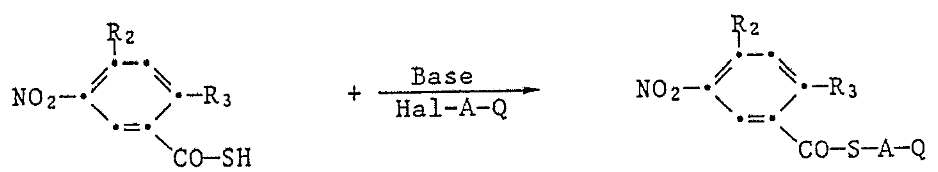


Der erhaltene 3-Nitrobenzoesäurethiolester wird dann mit Wasserstoff in Gegenwart eines Katalysators, z.B.

Raney Nickel zum 3-Amino-benzoesäureester reduziert. Das Amin wird anschliessend mit einem 3,4,5,6-Tetrahydrophthalsäureanhydrid kondensiert, entsprechend dem erfindungsgemässen Verfahren.

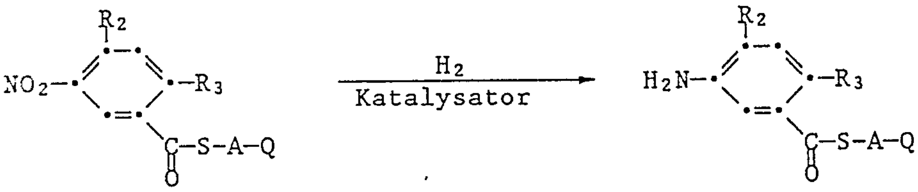


III

Arylthiocarbonsäuren resp. die Thiolbenzoesäure sind bekannt (Beilstein Bd. 9 419 I, 169; Org. Synthesis 37, 101) und können beispielsweise durch Umsetzung eines gegebenenfalls substituierten Benzoylchlorides mit Natriumhydrogensulfid hergestellt werden.

Thiolbenzoesäure kann z.B. hergestellt werden, indem man Benzoylchlorid in kalte, mit Schwefelwasserstoff gesättigte alkoholische Lösung von Kaliumhydroxyd einträgt, vom entstandenen Kaliumchlorid abfiltriert und das Filtrat zur Trockne einengt. Das erhaltene Kaliumsalz kann mit Salzsäure zersetzt werden, wobei Thiolbenzoesäure entsteht.

Ein anderer Weg zur Herstellung von Benzoesäurethiolestern ist in Tetrahedron Letters Vol. 27 (32) pp. 3791-3794 (1986) beschrieben. Gemäss dem kondensiert man ein Benzoesäurehalogenid in Gegenwart einer katalytischen Menge wasserfreiem Cobaltchlorides $Co^{II}Cl_2$ mit einem Merkaptan.

Gemäss einem weiteren erfindungsgemässen Verfahren erhält man die 5-(N-3,4,5,6-Tetrahydrophthalsäureimido)-benzoesäurethiolester der Formel I Anspruch 1, indem man ein 5-(N-3,4,5,6-Tetrayhydrophthalsäureimido)-benzoesäurehalogenid der Formel V

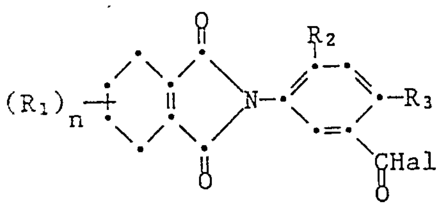

(V)

worin n, $R_1$, $R_2$ und $R_3$ die unter der Formel I gegebene Bedeutung haben und Hal ein Halogenatom, vorzugsweise Chlor oder Brom bedeutet, in einem inerten organischen Lösungs- oder Verdünnungsmittel in Gegenwart einer Base mit Schwefelwasserstoff und anschliessend mit einer reaktiven Verbindung der Formel VI umsetzt

X - A - Q (VI)

worin A und Q die unter der Formel I gegebene Bedeutung haben und X eine elektronegative Abgangsgruppe, z.B. Halogen oder ein Sulfonsäurerest bedeutet.

Als Lösungs- oder Verdünnungsmittel kommen auch für diese Umsetzung höhersiedende Kohlenwasserstoffe, niedere Ester von Alkansäuren, höhersiedende Ketone und Aether in Frage. Als Beispiel seien Hexan, Benzol, Toluol, Xylol, Essigsäureethylester, Isopropyläther, Dioxan, Methylethylketon erwähnt.

Die Umsetzung findet bei einer Temperatur statt, die zwischen 0°C und dem Siedepunkt des Reaktionsgemisches liegt.

Als Basen kommt tertiäre Amine, wie Triethylamin, Pyridin oder Collidin sowie die Hydroxide, Carbonate und Bicarbonate von Alkalimetallen in Frage.

Das 5-(N-3,4,5,6-Tetrahydrophthalsäureimido)-benzoesäurehalogenid der Formel V wird durch Kondensation eines Tetrahydrophthalsäureanhydrides der Formel II mit einer entsprechend durch $R_2$ und $R_3$ substituierten 3-Aminobenzoesäure und anschliessender Halogenierung gemäss dem beschriebenen Verfahren hergestellt.

Ein weiteres Verfahren zur Herstellung der 5-(N-3,4,5,6-Tetrahydrophthalsäureimido)-benzoesäurethiolester der Formel I besteht darin, dass man eine 5-(N-3,4,5,6-Tetrahydrophthalsäureimido)-benzoesäurehalogenid der Formel VI

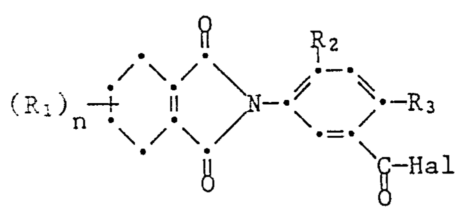

(VI)

worin n, R, $R_1$ und $R_2$ die unter Formel I gegebene Bedeutung haben und Hal für ein Halogenatom,

vorzugsweise Chlor oder Brom steht, in einem inerten organischen Lösungs- oder Verdünnungsmittel in Gegenwart der molaren Menge einer Base mit einem Merkaptan der Formel VII umsetzt

HS - A - Q (VII)

worin A und Q die im Anspruch 1 gegebene Bedeutung haben.

Als Lösungs- oder Verdünnungsmittel kommen die unter dem ersten oder zweiten Verfahren aufgezählten in Frage.

Die Umsetzung findet bei einer Temperatur statt, die zwischen 0°C und dem Siedepunkt des Reaktionsgemisches liegt.

Als Basen können auch hier die oben erwähnten tertiären Amine, Hydroxide, Carbonate oder Bicarbonate von Alkalimetallen eingesetzt werden.

Die Reaktionsbedingungen sind bei allen beschriebenen Verfahren ähnlich.

Die Wirkstoffe der Formel I werden in der Regel mit Aufwandmengen von 0,05 bis 4 kg/ha insbesondere 0,1 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Mais und Reis, vor allem aber Soja befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weitere Mechanismus der Ertragssteigerung mit Wachstumshemmern beruht darauf, dass die Nährstoffe in stärkerem Masse der Blüten-und Fruchtbildung zugute kommen, während das vegetative Wachstum eingeschränkt wird.

Die Wirkstoffe der Formel I können ferner zur Defoliation und Dessiccation von Baumwoll- und Kartoffelkulturen eingesetzt werden. Durch Behandeln der Kulturen im Zeitpunkt der Reife wird die Ernte der Baumwollkapseln oder der Knollen stark erleichtert, wenn die Blätter und Stauden abgefallen und/oder zur Dürre getrocknet sind.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B.

Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlensotffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1981. Dr. Helmut Stache "Tensid Taschenbuch" Carl Hanser Verlag, München/Wien 1981.

Die herbiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

| | | |
|---|---|---|
| Emulgierbare Konzentrate: | | |
| Wirkstoff der Formel I: | 1 bis 20 % bevorzugt | 5 bis 10 % |
| oberflächenaktives Mittel: | 5 bis 30 %, vorzugsweise | 10 bis 20 % |
| flüssiges Trägermittel: | 50 bis 94 %, vorzugsweise | 70 bis 85 %. |
| Stäube: | | |
| Wirkstoff der Formel I: | 0,1 bis 10 %, vorzugsweise | 0,1 bis 1 % |
| festes Trägermittel: | 99,9 bis 90 %, vorzugsweise | 99,9 bis 99 %. |
| Suspensions-Konzentrate: | | |
| Wirkstoff der Formel I: | 5 bis 75 %, vorzugsweise | 10 bis 50 % |
| Wasser: | 94 bis 25 %, vorzugsweise | 90 bis 30 % |
| oberflächenaktives Mittel: | 1 bis 40 %, vorzugsweise | 2 bis 30 %. |
| Benetzbare Pulver: | | |
| Wirkstoff der Formel I: | 0,5 bis 90 %, vorzugsweise | 1 bis 80 % |
| oberflächenaktives Mittel: | 0,5 bis 20 %, vorzugsweise | 1 bis 15 % |
| festes Trägermittel: | 5 bis 95 %, vorzugsweise | 15 bis 90 %. |
| Granulate: | | |
| Wirkstoff der Formel I: | 0,5 bis 30 %, vorzugsweise | 3 bis 15 % |
| festes Trägermittel: | 99,5 bis 70 %, vorzugsweise | 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele veranschaulichen die Herstellung von Verbindungen der Formel I. Weitere in entsprechender Weise hergestellte Wirkstoffe sind in den anschliessenden Tabellen aufgeführt. Temperaturangaben beziehen sich auf Celsius-Grade, Drucke sind in Millibar angegeben.

Beispiel 1: Herstellung von N-[4-Chlor-5-(1-cyanoäth-1-ylthiocarbonyl)-2-fluor)phenyl]-3,4,5,6-tetrahydrophthalsäureimid

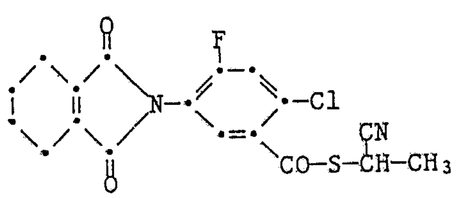

In 60 g Pyridin, welches auf -5°C gekühlt ist, trägt man, unter Rühren und gleichzeitigem Einleiten von Schwefelwasserstoff, portionenweise während 1/2 Stunde 6,8 g N-(4-chlor-5-chlorcarbonyl-2-fluorphenyl)-3,4,5,6-tetrahydrophthalsäureimid ein. Wenn alles zugegeben ist, nimmt man die Kühlung weg und rührt

während 2 Stunden bei Raumtemperatur weiter. Das Reaktionsgemisch wird anschliessend am Vakuum eingeengt. Der Rückstand wird in 50 ml Toluol aufgenommen und zuerst mit 6 ml Triethylamin, dann mit 2,7 g 2-Brompropionitril versetzt und 2 1/2 Stunden bei 50°C gerührt. Es entsteht ein Niederschlag, der abgenutscht wird. Das Filtrat wird eingeengt, in Wasser und Essigsäureethylester aufgenommen. Die wässrige Phasen wird verworfen und die organische Phase über Natriumsulfat getrocknet. Nach dem Einengen wird der Rückstand mittels Essigsäureethylester/Hexan 2:7 über eine Kieselgelsäule chromatographiert. Nach Verdampfen des Eluates verbleiben 5,3 g Titelprodukt als farbloses Oel $n_D^{20}$ 1,5709.

Beispiel 2:Herstellung von N-[4-Chlor-2-fluor-5-(triethoxysilylethylthiocarbonyl)phenyl]-3,4,5,6-tetrahydrophthalsäureimid

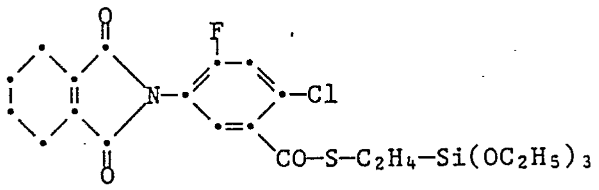

Zu einer Lösung von 5 ml Merkaptoethyltriethoxysilan und 4 ml Triethylamin in 150 ml Toluol wird unter Rühren bei 0-5°C eine Lösung von 6,8 g N-(4-Chlor-5-chlorcarbonyl-2-fluorphenyl)-3,4,5,6-tetrahydrophthalsäureimid getropft. Das Reaktionsgemisch wird 12 Stunden bei Raumtemperatur gerührt und dann mit 500 ml Wasser versetzt. Die organische Phase wird abgetrennt, über Magnesiumsulfat getrocknet und im Vakuum eingeengt. Es verbleibt ein braunes Oel, das durch Chromatographie mittels Essigsäureäthylester/Hexan 2:7 über eine Kieselgelsäure gereinigt wird. Nach Verdampfen des Lösungsmittels verbleiben 8,6 g Titelprodukt als farbloses Oel $n_D^{21}$ 1,5358.

In analoger Weise zu diesen Beispielen werden die Verbindungen der Tabellen 1 und 2 hergestellt.

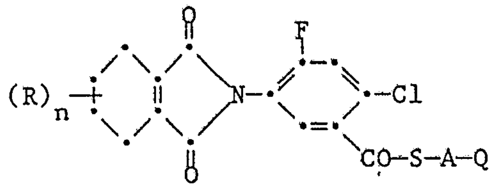

Tabelle 1

| Nr. | $(R)_n$ | A-Q | phys. Daten |
|---|---|---|---|
| 1.001 | - | $-CH(CH_3)CH_2OH$ | |
| 1.002 | - | $-CH(CH_3)CN$ | $n_D^{20}$ 1,5709 (Bsp. 1) |
| 1.003 | - | $-C_2H_4OH$ | Smp. 98-99° |
| 1.004 | - | $-CH(CH_3)CH_2Cl$ | |
| 1.005 | - | $-C_2H_4Cl$ | |
| 1.006 | - | $-CH_2CN$ | |
| 1.007 | - | $-CH_2C(CH_3)_2OH$ | |
| 1.008 | - | $-CH_2SCN$ | |
| 1.009 | - | $-C_2H_4CN$ | |
| 1.010 | - | $-CH(CH_3)CH_2CN$ | |
| 1.011 | - | $-CH(CH_2OCH_3)COOCH_3$ | $n_D^{24}$ 1,5372 |
| 1.012 | - | $-CH_2CH(OCH_3)COOCH_3$ | |
| 1.013 | - | $-CH(CH_3)COOC_2H_4OCH_2CH=CH_2$ | |
| 1.014 | - | $-CH(CN)CH_2OCH_3$ | |
| 1.015 | - | $-CH_2CH(SCH_3)COOCH_3$ | |
| 1.016 | - | $-CH_2CH=CH_2$ | |
| 1.017 | - | $-CH_2CH=CHCl$ | |
| 1.018 | - | $-CH_2CCl=CH_2$ | |
| 1.019 | - | $-CH_2CH=CHCN$ | |
| 1.020 | - | $-CH_2C\equiv CH$ | |
| 1.021 | - | $-CH_2C(CH_3)=CHCOOCH_3$ | |
| 1.022 | - | $-C_2H_4N(CH_3)_2$ | Smp. 76-78° |
| 1.023 | - | $-C_2H_4$-pyrrolidino | |
| 1.024 | - | $-C_2H_4$-piperidino | |
| 1.025 | - | $-C_2H_4$-morpholino | |
| 1.026 | - | $-CH[N(CH_3)_2]COOCH_3$ | |
| 1.027 | - | $-CH(CH_3)CH_2N(CH_3)_2$ | |
| 1.028 | - | $-CH(CH_3)CO$-piperidino | Harz |

| Nr. | $(R)_n$ | A-Q | phys. Daten |
|---|---|---|---|
| 1.029 | - | $-CH(CH_3)CO$-pyrrolidino | Smp. 57-60° |
| 1.030 | - | $-CH(CH_3)CO$-4-methylpiperazino | |
| 1.031 | - | $-CH(CH_3)CO$-morpholino | |
| 1.032 | - | $-CH(CH_3)CO$-2-methylmorpholino | $n_D^{20}$ 1,5342 |
| 1.033 | - | $-CH(CH_3)CO$-2,6-dimethylmorpholino | |
| 1.034 | - | $-CH(CH_3)CO$-2-methylpiperidino | |
| 1.035 | - | $-CH(CH_3)CO$-3-methylpiperidino | |
| 1.036 | - | $-CH(CH_3)CO$-4-methylpiperidino | |
| 1.037 | - | $-CH(CH_3)CO-N(CH_3)CH_2CN$ | |
| 1.038 | - | $-CH(CH_3)CO-N(CH_2CH=CH_2)_2$ | |
| 1.039 | - | $-CH(CH_3)CO-OCH_2Si(CH_3)_3$ | |
| 1.040 | - | $-CH(CH_3)CO-OC_2H_4Si(CH_3)_3$ | |
| 1.041 | - | $-CH(CH_3)CO-ON=C(CH_3)_2$ | |
| 1.042 | - | $-CH(CH_3)CO-ON=$cyclopentyl | |
| 1.043 | - | $-CH(CH_3)CO-ON=$cyclohexyl | |
| 1.044 | - | -1,3-dioxolan-2-yleth-1-yl | |
| 1.045 | - | -1,3-dioxolan-2-ylmethyl | Smp. 99-100° |
| 1.046 | - | -4-methyl-1,3-dioxolan-2-ylmethyl | |
| 1.047 | - | -1,3-dioxolan-2-ylpropyl | |
| 1.048 | - | -2-methyl-1,3-dioxolan-2-ylpropyl | |
| 1.049 | - | $-C_2H_4C(OCH_3)_2$ | |
| 1.050 | - | $-C_2H_4C(OC_2H_5)_2$ | |
| 1.051 | - | $-CH(CH_3)C(OC_2H_5)_2$ | |
| 1.052 | - | $-CH(CH_3)C(OCH_3)_2$ | |
| 1.053 | - | -2-methyl-1,3-dioxolan-2-ylmethyl | |
| 1.054 | - | $-C_2H_4Si(OC_2H_5)_3$ | $n_D^{21}$ 1,5358 (Bsp. 2) |
| 1.055 | - | $-CH_2Si(CH_3)_3$ | $n_D^{22}$ 1,5638 |
| 1.056 | - | $-CH(CH_3)Si(CH_3)_3$ | |
| 1.057 | - | $-C_2H_4Si(CH_3)_3$ | |
| 1.058 | - | $-CH(CH_3)CH_2Si(CH_3)_3$ | |
| 1.059 | - | $-CH(CH_3)PO(OC_2H_5)_2$ | |
| 1.060 | - | $-CH(CH_3)PO(CH_3)OC_2H_5$ | |
| 1.061 | - | $-CH(CH_3)CON(CH_3)SO_2CH_3$ | |
| 1.062 | - | $-CH(CH_3)CON(CH_3)SO_2CH_2Cl$ | |
| 1.063 | - | $-CH_2CON(CH_3)SO_2CH_3$ | |

| Nr. | $(R)_n$ | A-Q | phys. Daten |
|---|---|---|---|
| 1.064 | - | $-CH(CH_3)CON(CH_2CH=CH_2)SO_2CH_3$ | |
| 1.065 | - | $-CH(CH_3)CON$(cyclopropyl)$SO_2CH_3$ | |
| 1.066 | - | $-CH(CH_3)CON[CH(CH_3)_2]SO_2CH_3$ | |
| 1.067 | - | $-CH_2COCH_3$ | |
| 1.068 | - | $-CH_2CO$ benzyl | |
| 1.069 | - | $-CH_2CO$ phenyl | |
| 1.070 | - | $-CH_2COCH_2OCH_3$ | |
| 1.071 | - | $-CH(CH_3)COCH_3$ | |
| 1.072 | - | $-CH(CH_3)COCH_2COOCH_3$ | |
| 1.073 | - | $-CH(COCH_3)COOC_2H_5$ | |
| 1.074 | - | $-CH(CH_3)COOCH(CH_3)CH_2SCH_3$ | |
| 1.075 | - | $-CH(CH_3)COOCH(CH_3)CH_2SC_2H_5$ | $n_D^{21}$ 1,5620 |
| 1.076 | - | $-CH(CH_3)COOCH(CH_3)CH_2SC_3H_7-n$ | |
| 1.077 | - | $-CH(CH_3)COOCH(CH_3)CH_2SCH(CH_3)_2$ | |
| 1.078 | - | $-CH(CH_3)COOCH(CH_3)CH_2SC_4H_9-n$ | |
| 1.079 | - | $-CH(CH_3)COOCH(CH_3)CH_2SC(CH_3)_3$ | |
| 1.080 | - | $-CH(CH_3)COOCH(CH_3)CH_2SCH(CH_3)C_2H_5$ | |
| 1.081 | - | $-CH(CH_3)CON(CH_3)CH_2CH(OCH_3)_2$ | |
| 1.082 | - | $-CH(CH_3)CON(CH_3)CH_2CH(OC_2H_5)_2$ | |
| 1.083 | - | $-CH(CH_3)COOC_2H_4OCH_2CH=CH_2$ | |
| 1.084 | - | $-CH(COCH_3)_2$ | |
| 1.085 | - | $-CH(CN)COOCH_3$ | |
| 1.086 | - | $-CH(OCH_3)COOCH_3$ | Harz |

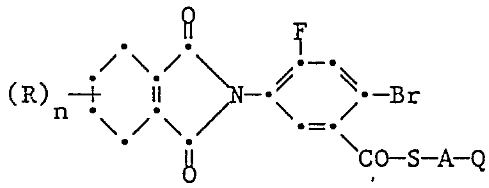

Tabelle 2

| Nr. | $(R)_n$ | A-Q | phys. Daten |
|---|---|---|---|
| 2.001 | - | $-CH(CH_3)CH_2OH$ | |
| 2.002 | - | $-CH(CH_3)CN$ | |
| 2.003 | - | $-C_2H_4OH$ | |
| 2.004 | - | $-CH(CH_3)CH_2Cl$ | |
| 2.005 | - | $-C_2H_4Cl$ | |
| 2.006 | - | $-CH_2CN$ | |
| 2.007 | - | $-CH_2C(CH_3)_2OH$ | |
| 2.008 | - | $-CH_2SCN$ | |
| 2.009 | - | $-C_2H_4CN$ | |
| 2.010 | - | $-CH(CH_3)CH_2CN$ | |
| 2.011 | - | $-CH(CH_2OCH_3)COOCH_3$ | |
| 2.012 | - | $-CH_2CH(OCH_3)COOCH_3$ | |
| 2.013 | - | $-CH(CH_3)COOC_2H_4OCH_2CH=CH_2$ | |
| 2.014 | - | $-CH(CN)CH_2OCH_3$ | |
| 2.015 | - | $-CH_2CH(SCH_3)COOCH_3$ | |
| 2.016 | - | $-CH_2CH=CH_2$ | |
| 2.017 | - | $-CH_2CH=CHCl$ | |
| 2.018 | - | $-CH_2CCl=CH_2$ | |
| 2.019 | - | $-CH_2CH=CHCN$ | |
| 2.020 | - | $-CH_2C\equiv CH$ | |
| 2.021 | - | $-CH_2C(CH_3)=CHCOOCH_3$ | |
| 2.022 | - | $-C_2H_4N(CH_3)_2$ | |
| 2.023 | - | $-C_2H_4$-pyrrolidino | |
| 2.024 | - | $-C_2H_4$-piperidino | |
| 2.025 | - | $-C_2H_4$-morpholino | |
| 2.026 | - | $-CH[N(CH_3)_2]COOCH_3$ | |
| 2.027 | - | $-CH(CH_3)CH_2N(CH_3)_2$ | |
| 2.028 | - | $-CH(CH_3)CO$-piperidino | |

| Nr. | $(R)_n$ | A-Q | phys. Daten |
|---|---|---|---|
| 2.029 | - | $-CH(CH_3)CO$-pyrrolidino | |
| 2.030 | - | $-CH(CH_3)CO$-4-methylpiperazino | |
| 2.031 | - | $-CH(CH_3)CO$-morpholino | |
| 2.032 | - | $-CH(CH_3)CO$-2-methylmorpholino | |
| 2.033 | - | $-CH(CH_3)CO$-2,6-dimethylmorpholino | |
| 2.034 | - | $-CH(CH_3)CO$-2-methylpiperidino | |
| 2.035 | - | $-CH(CH_3)CO$-3-methylpiperidino | |
| 2.036 | - | $-CH(CH_3)CO$-4-methylpiperidino | |
| 2.037 | - | $-CH(CH_3)CO-N(CH_3)CH_2CN$ | |
| 2.038 | - | $-CH(CH_3)CO-N(CH_2CH=CH_2)_2$ | |
| 2.039 | - | $-CH(CH_3)CO-OCH_2Si(CH_3)_3$ | |
| 2.040 | - | $-CH(CH_3)CO-OC_2H_4Si(CH_3)_3$ | |
| 2.041 | - | $-CH(CH_3)CO-ON=C(CH_3)_2$ | |
| 2.042 | - | $-CH(CH_3)CO-ON=$cyclopentyl | |
| 2.043 | - | $-CH(CH_3)CO-ON=$cyclohexyl | |
| 2.044 | - | -1,3-dioxolan-2-yleth-1-yl | |
| 2.045 | - | -1,3-dioxolan-2-ylmethyl | |
| 2.046 | - | -4-methyl-1,3-dioxolan-2-ylmethyl | |
| 2.047 | - | -1,3-dioxolan-2-ylpropyl | |
| 2.048 | - | -2-methyl-1,3-dioxolan-2-ylpropyl | |
| 2.049 | - | $-C_2H_4C(OCH_3)_2$ | |
| 2.050 | - | $-C_2H_4C(OC_2H_5)_2$ | |
| 2.051 | - | $-CH(CH_3)C(OC_2H_5)_2$ | |
| 2.052 | - | $-CH(CH_3)C(OCH_3)_2$ | |
| 2.053 | - | -2-methyl-1,3-dioxolan-2-ylmethyl | |
| 2.054 | - | $-C_2H_4Si(OC_2H_5)_3$ | |
| 2.055 | - | $-CH_2Si(OH_3)_3$ | |
| 2.056 | - | $-CH(CH_3)Si(CH_3)_3$ | |
| 2.057 | - | $-C_2H_4Si(CH_3)_3$ | |
| 2.058 | - | $-CH(CH_3)CH_2Si(CH_3)_3$ | |
| 2.059 | - | $-CH(CH_3)PO(OC_2H_5)_2$ | |
| 2.060 | - | $-CH(CH_3)PO(CH_3)OC_2H_5$ | |
| 2.061 | - | $-CH(CH_3)CON(CH_3)SO_2CH_3$ | |
| 2.062 | - | $-CH(CH_3)CON(CH_3)SO_2CH_2Cl$ | |
| 2.063 | - | $-CH_2CON(CH_3)SO_2CH_3$ | |

| Nr. | $(R)_n$ | A-Q | phys. Daten |
|---|---|---|---|
| 2.064 | - | $-CH(CH_3)CON(CH_2CH=CH_2)SO_2CH_3$ | |
| 2.065 | - | $-CH(CH_3)CON(cyclopropyl)SO_2CH_3$ | |
| 2.066 | - | $-CH(CH_3)CON[CH(CH_3)_2]SO_2CH_3$ | |
| 2.067 | - | $-CH_2COCH_3$ | |
| 2.068 | - | $-CH_2CO$ benzyl | |
| 2.069 | - | $-CH_2CO$ phenyl | |
| 2.070 | - | $-CH_2COCH_2OCH_3$ | |
| 2.071 | - | $-CH(CH_3)COCH_3$ | |
| 2.072 | - | $-CH(CH_3)COCH_2COOCH_3$ | |
| 2.073 | - | $-CH(COCH_3)COOC_2H_5$ | |
| 2.074 | - | $-CH(CH_3)COOCH(CH_3)CH_2SCH_3$ | |
| 2.075 | - | $-CH(CH_3)COOCH(CH_3)CH_2SC_2H_5$ | |
| 2.076 | - | $-CH(CH_3)COOCH(CH_3)CH_2SC_3H_7\text{-n}$ | |
| 2.077 | - | $-CH(CH_3)COOCH(CH_3)CH_2SCH(CH_3)_2$ | |
| 2.078 | - | $-CH(CH_3)COOCH(CH_3)CH_2SC_4H_9\text{-n}$ | |
| 2.079 | - | $-CH(CH_3)COOCH(CH_3)CH_2SC(CH_3)_3$ | |
| 2.080 | - | $-CH(CH_3)COOCH(CH_3)CH_2SCH(CH_3)C_2H_5$ | |
| 2.081 | - | $-CH(CH_3)CON(CH_3)CH_2CH(OCH_3)_2$ | |
| 2.082 | - | $-CH(CH_3)CON(CH_3)CH_2CH(OC_2H_5)_2$ | |
| 2.083 | - | $-CH(CH_3)COOC_2H_4OCH_2CH=CH_2$ | |
| 2.084 | - | $-CH(COCH_3)_2$ | |
| 2.085 | - | $-CH(CN)COOCH_3$ | |
| 2.086 | - | $-CH(CH_3)COOCH_3$ | |

Formulierungsbeispiele:

Beispiel 2: Formulierungsbeispiele für Wirkstoffe der Formel I (% = Gewichtsprozent)

| a) Spritzpulver | a) | b) | c) |
|---|---|---|---|
| Wirkstoff gemäss Tabellen 1 + 2 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |

| | a) | b) | c) |
|---|---|---|---|
| Wirkstoff gemäss Tabellen 1 + 2 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen

b) Emulsions-Konzentrat

| | a) | b) |
|---|---|---|
| Wirkstoff gemäss Tabellen 1 + 2 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff gemäss Tabellen 1 + 2 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff gemäss Tabellen 1 + 2 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff gemäss Tabellen 1+ 2 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) Suspensions-Konzentrat

| | a) | b) |
|---|---|---|
| Wirkstoff gemäss Tabellen 1 + 2 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige formaldehyd-Lösung | 0,2 % | 0,2 % |
| Silikonöl in Form einer 75%igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff gemäss Tabellen 1 + 2 | 5 % |
| Isopropylamin | 1 % |
| Oxtylphenolpolyäthylenglykoläther (78 Mol AeO) | 3 % |
| Wasser | 91 % |

Biologische Beispiele:

Beispiel 3: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet. Der Zustand der Pflanzen wurde gemäss folgender Notenskala boniliert

1 Pflanze abgestorben oder nicht zum Keimen gekommen

2-4 starke, graduell abnehmende Schädigung

5 mittlere Schädigung

6-8 leichtere Schadstufen

9 Pflanze wächst normal, wie unbehandelte Kontrollpflanzen.

Die Resultate sind in der Tabelle 3 festgehalten.

Tabelle 3

| Verbindung Aufwandmenge g/ha | 1.055 | | 1.086 | |
|---|---|---|---|---|
| | 2000 | 1000 | 2000 | 1000 |
| Pflanze | | | | |
| Gerste | 9 | 9 | 9 | 9 |
| Weizen | 9 | 9 | 9 | 9 |
| Mais | 9 | 9 | 9 | 9 |
| Hirse | 9 | 9 | 9 | 9 |
| Soja | 9 | 9 | 9 | 9 |
| Baumwolle | 9 | 9 | 9 | 9 |
| Sonnenblume | 9 | 9 | 9 | 9 |
| Amaranthus retroflexus | 1 | 2 | 4 | 4 |
| Chenopodum album | 1 | 4 | 1 | 4 |
| Solanum nigrum | 1 | 1 | 3 | 4 |
| Chrysanthemum leucum | 1 | 1 | 1 | 1 |
| Viola tricolcr | 1 | 2 | 2 | 4 |
| Veronic sp | 1 | 1 | 1 | 1 |

Beispiel 4: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Die Resultate sind in der Tabelle 4 zusammengefasst.

Tabelle 4

| Verbindung | 1.002 | | 1.003 | | 1.011 | | 1.032 | | 1.045 | | 1.054 | | 1.055 | | 1.086 | |
|---|---|---|---|---|---|---|---|---|---|---|---|---|---|---|---|---|
| Aufwandmenge g/ha | 1000 | 500 | 1000 | 500 | 1000 | 500 | 1000 | 500 | 1000 | 500 | 1000 | 500 | 1000 | 500 | 1000 | 500 |
| Pflanze | | | | | | | | | | | | | | | | |
| Gerste | 6 | 8 | 9 | 9 | 6 | 7 | 8 | 8 | 7 | 7 | 8 | 8 | 7 | 8 | 8 | 8 |
| Weizen | 6 | 7 | 8 | 9 | 7 | 8 | 8 | 8 | 8 | 9 | 8 | 8 | 8 | 8 | 8 | 8 |
| Mais | 6 | 7 | 8 | 8 | 7 | 8 | 6 | 7 | 6 | 7 | 7 | 8 | 9 | 9 | 5 | 6 |
| Hirse | 5 | 7 | 8 | 8 | 6 | 7 | 7 | 8 | 7 | 7 | 8 | 8 | 8 | 8 | 8 | 8 |
| Abutilon | 1 | 1 | 3 | 4 | 1 | 1 | 1 | 4 | 1 | 1 | 1 | 2 | 1 | 3 | 2 | 2 |
| Xanthium | 1 | 1 | 2 | 4 | 1 | 1 | 4 | 4 | 4 | 4 | 1 | 3 | 4 | 4 | 4 | 4 |
| Solanum nigrum | 1 | 1 | 2 | 3 | 3 | 4 | 1 | 1 | 1 | 3 | 1 | 1 | 4 | 4 | 2 | 2 |
| Ipomoea purpurea | 1 | 1 | 3 | 4 | 1 | 1 | 4 | 4 | 1 | 2 | 1 | 1 | 2 | 4 | 4 | 4 |
| Viola tricolor | 1 | 1 | 4 | 4 | 1 | 1 | 1 | 2 | 1 | 1 | 1 | 2 | 2 | 2 | 3 | 4 |

Beispiel 5: Selektive Herbizidwirkung in Soja

Sojapflanzen sowie Gräser und dikotyle Unkräuter werden im Gewächshaus angezogen bis sie nach ca. 2 Wochen das 4-Blatt Stadium erreicht haben. Dann werden sie mit einer verdünnten Wirkstoffbrühe gespritzt. Die behandelten Pflanzen werden im Gewächshaus bei optimalen Wuchsbedingungen, 26-28°C, 43-60 % relative Luftfeuchtigkeit, regelmässiges Bewässern, gehalten. Der Versuch wird 21 Tage nach der Behandlung ausgewertet und der Zustand der Pflanzen gemäss der obigen Notenskala ausgewertet. Die Resultate sind in der Tabelle 5 zusammengefasst.

Tabelle 5

| Verbindung | 1.002 | | | 1.032 | | | 1.045 | | | 1.054 | | |
|---|---|---|---|---|---|---|---|---|---|---|---|---|
| Aufwandmenge g/ha | 1000 | 500 | 250 | 1000 | 500 | 250 | 1000 | 500 | 250 | 1000 | 500 | 250 |
| Pflanze | | | | | | | | | | | | |
| Soja | 6 | 6 | 7 | 6 | 7 | 8 | 7 | 7 | 8 | 8 | 8 | 8 |
| Abutilon | 1 | 1 | 2 | 1 | 1 | 4 | 1 | 1 | 1 | 1 | 2 | 4 |
| Sida spinosa | 1 | 1 | 2 | 3 | 4 | 4 | 1 | 4 | 4 | 3 | 4 | 4 |
| Xanthium sp | 1 | 1 | 1 | 4 | 4 | 4 | 4 | 4 | 4 | 1 | 3 | 4 |
| Chenopodium album | 2 | 3 | 4 | 4 | 4 | 4 | 1 | 2 | 4 | 2 | 2 | 4 |
| Solanum nigrum | 1 | 1 | 1 | 1 | 1 | 1 | 1 | 3 | 4 | 1 | 1 | 1 |
| Ipomoea purpurea | 1 | 1 | 1 | 4 | 4 | 4 | 1 | 2 | 4 | 1 | 1 | 4 |
| Sinapis alba | 1 | 3 | 4 | 3 | 4 | 4 | 1 | 3 | 4 | 1 | 4 | 4 |
| Viola tricolor | 1 | 1 | 1 | 1 | 1 | 2 | 1 | 1 | 1 | 1 | 2 | 4 |
| Veronica sp | 1 | 1 | 1 | 4 | 4 | 4 | 2 | 2 | 4 | 2 | 4 | 4 |

Beispiel 6: Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 1/1) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20°C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen bewertet. Die geprüften Verbindungen der Tabellen 1 und 2 zeigen dabei gute bis sehr gute Herbizidwirkung.

Beispiel 7 Herbizidwirkung für Wasserreis (paddy)

Reis und die Wasserunkräuter Scirpus sp und Monocharia vag. werden in Plastikbechern (60 cm$^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die daher verwendete Dosis entspricht einer Wirkstoffmenge von 0,5 kg Wirkstoff pro Hektar (Spritzbrühmenge = 550 l/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Der Zustand der Pflanzen wird nach der oben gegebenen Notenskala verwertet. Die Resultate sind in der Tabelle 6 zusammengefasst.

Tabelle 6

| Pflanze Aufwandmenge g/ha | Reis | | Scirpus sp | | Monocharia vaginalis | |
|---|---|---|---|---|---|---|
| | 2000 | 1000 | 2000 | 1000 | 2000 | 1000 |
| Verbindung | | | | | | |
| 1.011 | 6 | 8 | 1 | 4 | 1 | 1 |
| 1.045 | 6 | 7 | 1 | 1 | 1 | 1 |
| 1.054 | 7 | 9 | 1 | 3 | 1 | 1 |
| 1.055 | 8 | 9 | 2 | 4 | 1 | 2 |

Beispiel 8: Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen des Wirkstoffes No. 1 in einer Aufwandmenge, die 1,2, 0,6 und 0,3 kg/ha im Feld entspräche, gespritzt. Unbehandelte Pflanzen wurden als Kontrolle belassen. Die Auswertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter).

In diesem Versuch beliessen die geprüften Verbindungen der Tabellen 1 und 2 bei Aufwandmengen von 0,6 und 1,2 kg/ha nach 7 Tagen bloss noch wenige vertrocknete Blätter auf den Pflanzen ($>$ 80 % Blattfall und Austrocknung).

Beispiel 9: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoffe als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabellen 1 und 2 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 10: Wuchsregulierung und Ertragssteigung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Tempeaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemässen Wirkstoffe der Tabellen 1 und 2 eine merkliche Erhöhung der Anzahl und des Gewichts der Schoten im Haupttrieb.

Beispiel 11: Wuchshemmung bei Getreide

In Kunststofftöpfen mit sterilisierter Erde werden in Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabellen 1 und 2 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 12: Wuchshemmung bie Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgelaufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 1 und 2 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabellen 1 und 2 bewirken eine Reduzierung des Neuzuwachses von um 10-30 % im Vergleich zur unbehandelten Kontrolle.

**Patentansprüche**

1. 5-(N-3,4,5,6-Tetrahydrophthalsäureimido)-benzoesäurethiolester der Formel I

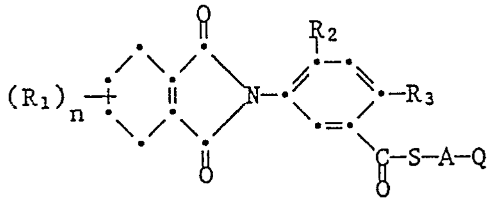

(I),

worin
n Null, eins oder zwei,
$R_1$ $C_1$-$C_3$-Alkyl,
$R_2$ Wasserstoff oder Halogen,
$R_3$ Halogen,
A-Q gemeinsam Wasserstoff oder ein Rest -$CH(COCH_3)COOR_4$),
A eine geradkettige oder verzweigte $C_1$-$C_4$-Alkylenbrücke, die unsubstituiert oder ein- oder mehrfach durch $C_1$-$C_4$-Alkoxy, $C_1$-$C_4$-Alkylthio oder -CN substituiert ist,
Q Hydroxyl, Halogen, -CN, -SCN, $C_2$-$C_6$-Alkenyl, $C_2$-$C_6$-Haloalkenyl, $C_2$-$C_6$-Cyanoalkenyl, $C_2$-$C_4$-Alkinyl oder einen Rest -$C(R_4)$=$CH$-$COOR_5$, -$CH[N(R_4)_2]COOR_4$, -$NR_6R_7$, -$COR_8$, -$CON(R_6)SO_2CH_3$, -$N(R_4)CH_2CN$, Diallylcarbamoyl, -$Si(R_{11})_3$, -$COOCH_2Si(CH_3)_2C_1$-$C_6$-alkyl, -$COON$=$C(R_9)_2$, -$C(R_4)$=$(OR_{10})_2$, -$PO(OR_{12})$-$(O)_mR_{12}$, -$CON(R_4)SO_2C_1$-$C_6$-alkyl, -$CON(R_4)$-$SO_2C_1$-$C_4$-Haloalkyl, $C_1$-$C_6$-Alkylcarbonyl, $C_2$-$C_6$-Alkoxyalkylcarbonyl, einen Benzoyl-oder Benzylcarbonylrest der unsubstituiert oder durch Halogen, $C_1$-$C_4$-Alkyl, $C_1$-$C_4$-Haloalkyl, $C_1$-$C_4$-Alkoxy, $C_1$-$C_4$-Haloalkoxy, Cyan oder Nitro ein- oder mehrfach substituiert ist, ferner einen Rest -$COOH(CH_3)CH_2SR_4$, -$CON(R_4)CH_2C(OC_1$-$C_6$-Alkyl$)_2$, -$COO(CH_2)_{n+1}OC_3$-$C_7$-Alkenyl, -$COOC_1$-$C_4$-Cyanoalkyl-$COOC_1$-$C_4$-AlkylenN$(R_4)_2$ oder $C_1$-$C_8$-Alkanoyloxyrest,
Q bedeutet auch einen Rest -$COOR_{13}$, wenn A durch $C_1$-$C_4$-Alkoxy, $C_1$-$C_4$-Alkylthio oder Cyano substituiert ist,
$R_4$ Wasserstoff, $C_{16}$-Alkyl oder $C_2$-$C_6$Alkoxyalkyl,
$R_5$ $C_1$-$C_6$-Alkyl oder $C_1$-$C_6$-Hydroxyalkyl,
$R_6$ und $R_7$ unabhängig voneinander je Wasserstoff, $C_1$-$C_6$-Alkyl, $C_2$-$C_6$-Alkoxyalkyl, $C_3$-$C_6$-Alkenyl, $C_3$-$C_6$-Alkinyl oder $C_3$-$C_6$-Cycloalkyl, 2-Furanylmethyl, 2-Tetrahydrofuranylmethyl, 2-(5-Methyl)-tetrahydrofuran ylmethyl, 2-Thienylmethyl,
$R_8$ einen Pyrrolidino-, Piperidino-, Morpholino-, Thiomorpholino-, 4-Methylpiperazino-, Pyrazolino-, Imidazolino- oder 1,2,4-Triazolinrest der unsubstituiert oder ein- oder zweifach durch $C_1$-$C_4$-Alkyl substituiert ist,
$R_9$ unabhängig voneinander je $C_1$-$C_6$-Alkyl oder zusammen eine $C_3$-$C_7$-Cycloalkyl
$R_{10}$ unabhängig voneinander $C_1$-$C_4$-Alkyl oder $C_1$-$C_4$-Haloalkyl oder zusammen eine 1,2-Aethylen-, 1,3-Propylen- oder 1,2-Cyclohexylenbrücke,
$R_{11}$ unabgängig voneinander $C_1$-$C_6$-Alkyl oder $C_1$-$C_6$-Alkoxy,
$R_{12}$ unabhängig voneinander Wasserstoff, $C_1$-$C_6$-Alkyl, $C_1$-$C_6$-Haloalkyl, $C_1$-$C_6$-Cyanoalkyl, $C_3$-$C_4$-Alkenyl oder $C_3$-$C_4$-Alkinyl,
$R_{13}$ $C_1$-$C_{12}$-Alkyl, $C_2$-$C_6$-Alkoxyalkyl, $C_3$-$C_7$-Alkenyl oder $C_3$-$C_7$-Cycloalkyl, und
m 0 oder 1
bedeuten sowie alle möglichen Stereoisomeren die in Form von Enantiomeren, Diastereomeren oder deren Gemische vorliegen.

2. 5-(N-3,4,5,6-Tetrahydrophthalsäureimido)-benzoesäurethiolester gemäss Anspruch 1 der Formel Ia

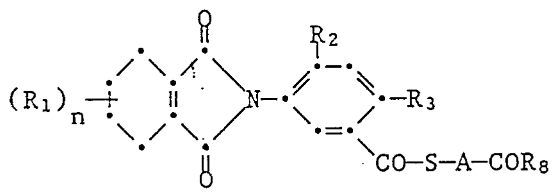

(Ia)

worin n Null, 1 oder 2, $R_1$ $C_3$-$C_6$-Alkyl, $R_2$ Fluor, $R_3$ Chlor oder Brom, A eine $C_1$-$C_4$-Alkylenkette und $R_8$ einen Pyrrolidino-, Piperidino-, Morpholino-, Thiomorpholino-, 4-Methylpiperazino-, Pyrazolino-, Imidazolidino-oder 1,2,4-Triazolinorest bedeutet, der unsubstituiert oder ein- oder zweifach durch $C_1$-$C_4$-Alkyl substituiert ist, bedeuten.

3. N-[4-Chlor-2-fluor-5-(2-methylmorpholinocarbonyl-eth-1-ylthiocarbonyl)phenyl]-3,4,5,6-tetrahydrophthalimid gemäss Anspruch 1.

4. N-[4-Chlor-2-fluor-5-(pyrrolidinocarbonyl-eth-1-ylthiocarbonyl)phenyl]-3,4,5,6-tetrahydrophthalimid gemäss Anspruch 1.

5. N-[4-Chlor-2-fluor-5-(piperidinocarbonyl-eth-1-ylthiocarbonyl)phenyl]-3,4,5,6-tetrahydrophthalimid gemäss Anspruch 1

6. 5-(N-3,4,5,6-Tetrahydrophthalsäureimido)-benzoesäurethiolester gemäss Anspruch 1 der Formel Ib

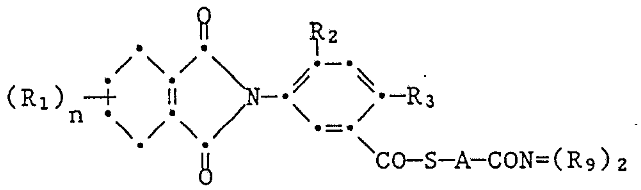

(Ib)

worin n Null, 1 oder 2, $R_1$ $C_1$-$C_3$-Alkyl, $R_2$ Fluor, $R_3$ Chlor oder Brom, A eine $C_1$-$C_4$-Alkylenbrücke und die $R_9$ unabhängig voneinander je $C_1$-$C_6$-Alkyl oder zusammen eine $C_3$-$C_7$-Cycloalkyl bedeuten.

7. N-[5-(Acetonoxim-carbonyleth-1-ylthiocarbonyl-2-fluor-4-chlor)phenyl]-3,4,5,6-tetrahydrophthalimid gemäss Anspruch 1.

8. 5-(N-3,4,5,6-Tetrahydrophthalsäureimido)-benzoesäurethiolester gemäss Anspruch 1 der Formel Ic

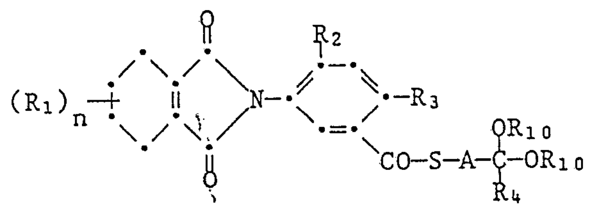

(Ic)

worin n Null, 1 oder 2, $R_1$ $C_3$-$C_6$-Alkyl, $R_2$ Fluor, $R_3$ Chlor oder Brom, A eine $C_1$-$C_4$-Alkylenbrücke, $R_4$ Wasserstoff, $C_1$-$C_6$-Alkyl oder $C_2$-$C_6$-Alkoxyalkyl und die $R_{10}$ unabhängig voneinander je $C_1$-$C_4$-Alkyl, $C_1$-$C_4$-Haloalkyl oder zusammen eine 1,2-Aethylen-, 1,3-Propylen- oder 1,2-Cyclohexylenbrücke bedeuten.

9. N-[4-Chlor-5-(1,3-dioxolan-2-ylmethylthiocarbonyl-2-fluor)phenyl]-3,4,5,6-tetrahydrophthalimid gemäss Anspruch 1.

10. 5-(N-3,4,5,6-Tetrahydrophthalsäureimido)-benzoesäurethiolester gemäss Anspruch 1 der Formel Id

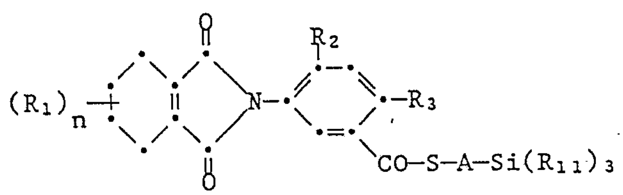

(Id)

worin n Null, 1 oder 2, $R_1$ $C_3$-$C_6$-Alkyl, $R_2$ Fluor, $R_3$ Chlor oder Brom, A eine $C_1$-$C_4$-Alkylenbrücke und die $R_{11}$ unabhängig voneinander je $C_1$-$C_6$-Alkyl oder $C_1$-$C_6$-Alkoxy bedeuten.

11. N-[4-Chlor-2-fluor-5-(triethoxysilyleth-2-ylthiocarbonyl)phenyl]-3,4,5,6-tetrahydrophthalimid gemäss Anspruch 1.

12. N-[4-Chlor-2-fluor-5-(trimethylsilylmethylthiocarbonyl)phenyl]-3,4,5,6-tetrahydrophthalimid gemäss Anspruch 1.

13. 5-(N-3,4,5,6-Tetrahydrophthalsäureimido)-benzoesäurethiolester gemäss Anspruch 1 der Formel If

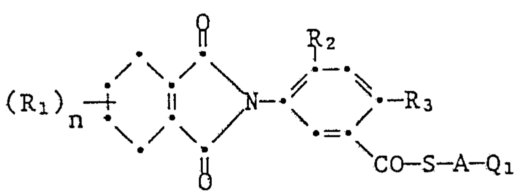

(If)

worin A, n, $R_1$, $R_2$ und $R_3$ die in Anspruch 1 gegebene Bedeutung haben und $Q_1$ Hydroxyl oder Cyano bedeutet.

14. N-[4-Chlor-2-fluor-5-(1-cyano-eth-1-ylthiocarbonyl)phenyl]-3,4,5,6-tetrahydrophthalimid gemäss Anspruch 1.

15. 5-[4-Chlor-2-fluor-5-(hydroxyethylthiocarbonyl)phenyl-3,4,5,6-tetrahydrophthalimid gemäss Anspruch 1.

16. 5-(N-3,4,5,6-Tetrahydrophthalsäureimido)-benzoesäurethiolester gemäss Anspruch 1 der Formel Ii

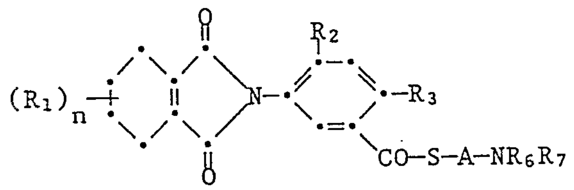

(Ii)

worin A, n, $R_1$, $R_2$, $R_3$, $R_6$ und $R_7$ die in Anspruch 1 gegebene Bedeutung haben.

17. N-[4-Chlor-5-(dimethylaminoethylthiocarbonyl)-2-fluorophenyl]-3,4,5,6-tetrahydrophthalimid gemäss Anspruch 1.

18. 5-(N-3,4,5,6-Tetrahydrophthalsäureimido)-benzoesäurethiolester gemäss Anspruch 1 der Formel Ij

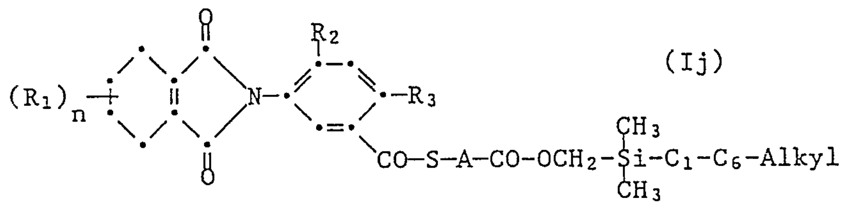

(Ij)

worin A, n, $R_1$, $R_2$ und $R_3$ die in Anspruch 1 gegebene Bedeutung haben.

19. 5-(N-3,4,5,6-Tetrahydrophthalsäureimido)-benzoesäurethiolester gemäss Anspruch 1 der Formel Il

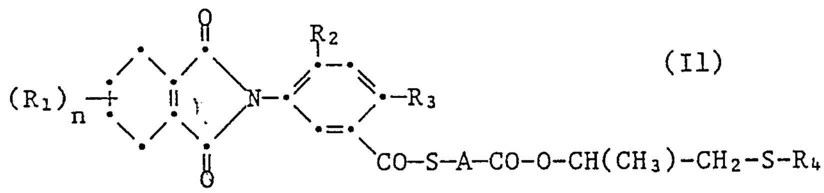

(Il)

worin A, n, $R_1$, $R_2$ und $R_3$ die in Anspruch 1 gegebene Bedeutung haben und $R_4$ $C_1$-$C_6$-Alkyl oder $C_2$-$C_6$-Alkoxyalkyl bedeutet.

20. N-[5-(Aethylthioprop-2-yloxycarbonyleth-1-ylthiocarbonyl)-4-chlor-2-fluor-phenyl]-3,4,5,6-tetrahydrophthalimid gemäss Anspruch 1.

21. 5-(N-3,4,5,6-Tetrahydrophthalsäureimido)-benzoesäurethiolester gemäss Anspruch 1 der Formel In

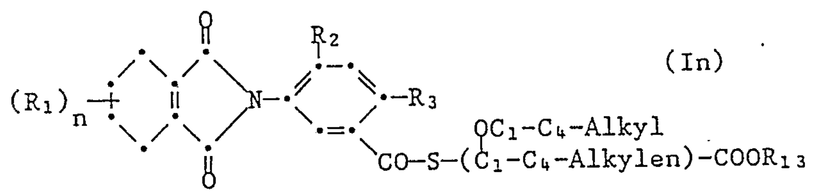

(In)

worin n, $R_1$, $R_2$, $R_3$ und $R_{13}$ die in Anspruch 1 gegebene Bedeutung haben.

22. N-[4-Chlor-2-fluor-5-(1′-methoxycarbonyl-2′-methoxymethyl-ethylthiocarbonyl)phenyl]-3,4,5,6-tetrahydrophthalimid gemäss Anspruch 1.

23. N-[4-Chlor-2-fluor-5-(1′-methoxycarbonyl-2′methoxymethyl-ethylthiocarbonyl)phenyl]-3,4,5,6-tetrahydrophthalimid gemäss Anspruch 1.

24. Verfahren zur Herstellung der 5-(N-3,4,5,6-tetrahydrophthalsäureimid)-benzoesäurethiolester der Formel I, dadurch gekennzeichnet, dass man das Anhydrid einer 3,4,5,6-Tetrahydrophthalsäure der Formel II

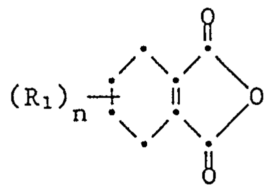

(II)

worin $R_1$ und n die unter der Formel I gegebene Bedeutung haben, in einem inerten organischen Lösungs- oder Verdünnungsmittel, gegebenenfalls in Gegenwart einer geringen Menge eines wasserentziehenden Mittels, mit der äquimolaren Menge eines Derivates einer 3-Aminobenzoesäureester der Formel III umsetzt

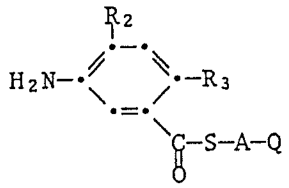

(III),

worin A, $R_2$ und $R_3$ die unter der Formel I gegebene Bedeutung haben.

25. Verfahren zur Herstellung des 5-(N-3,4,5,6-Tetrahydrophthalsäureimido)-benzoesäurethiolester der Formel I Anspruch 1, dadurch gekennzeichnet, dass man ein 5-(N-3,4,5,6-Tetrahydrophthalsäureimido)benzoesäurehalogenid der Formel V

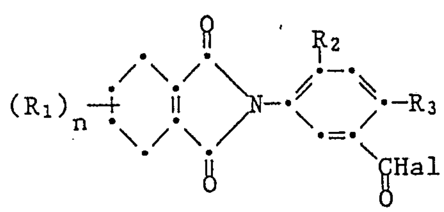

(V)

worin n, $R_1$, $R_2$ und $R_3$ die unter der Formel I gegebene Bedeutung haben und Hal ein Halogenatom, vorzugsweise Chlor oder Brom bedeutet, in einem inerten organischen Lösungs- oder Verdünnungsmittel in Gegenwart einer Base mit Schwefelwasserstoff und anschliessend mit einer reaktiven Verbindung der Formel VI umsetzt

X - A - Q (VI)

worin A und Q die unter der Formel I gegebene Bedeutung haben und X eine elektronegative Abgangsgruppe, z.B. Halogen oder ein Sulfonsäurerest bedeutet.

26. Ein herbizides und Pflanzenwachstum regulierendes Mittel, dadurch gekennzeichnet, dass es neben Träger- und/oder anderen Zuschlagstoffen als Wirkstoff ein 5-(N-3,4,5,6-Tetrahydrophthalsäureimido)-benzoesäureester der Formel I, Anspruch 1 enthält.

27. Verfahren zur selektiven pre- oder postemergenten Bekämpfung von Unkräutern in Nutzpflanzenkulturen, dadurch gekennzeichnet, dass man die Nutzpflanzenkulturen oder deren Anbaufläche mit einer wirksamen Menge eines Wirkstoffes der Formel I, Anspruch 1 oder eines eine solche Verbindung enthaltenden Mittels behandelt.

28. Verfahren zur Unterdrückung der Pflanzenwachstums über das 2-Blattstadium hinaus, dadurch gekennzeichnet, dass man die Pflanzen während ihres Wachstums mit einer wirksamen Menge eines Wirkstoffes der Formel I Anspruch 1 oder eines einen solchen Wirkstoff enthaltendes Mittel behandelt.

29. Verfahren zur Defoliation und Dessiccation von Baumwoll- und Kartoffelkulturen zum Erleichtern der Ernte, dadurch gekennzeichnet, dass man die Kultur kurz vor der Ernte mit einer wirksamen Menge eines Wirkstoffes gemäss Anspruch 1 oder eines eine solche Verbindung enthaltendes Mittels behandelt.

Europäisches Patentamt

# EUROPÄISCHER RECHERCHENBERICHT

Nummer der Anmeldung

EP 89810280.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | DE - A1 - 3 109 035 (NIPPON KAYAKU K.K.) * Patentanspruch 1; Seiten 13,15,17,22,26 * -- | 1,24-27 | C 07 D 209/48<br>C 07 D 405/12<br>C 07 F 7/10<br>C 07 F 7/18<br>A 01 N 43/00 |
| D,A | EP - A2/A3 - 0 233 151 (CIBA-GEIGY AG) * Patentansprüche 1,43,44,46, 50-52 * -- | 1,24-29 | |
| D,A | EP - A1 - 0 207 894 (CIBA-GEIGY AG) * Patentansprüche 1,35,36,39, 43,44 * ---- | 1,24-28 | |

RECHERCHIERTE SACHGEBIETE (Int. Cl.4)

C 07 D 209/00
C 07 D 405/00
C 07 F 7/00

Der vorliegende Recherchenbericht wurde für alle Patentansprüche erstellt.

| Recherchenort | Abschlußdatum der Recherche | Prüfer |
|---|---|---|
| WIEN | 28-06-1989 | HEIN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument